(19) Europäisches Patentamt

European Patent Office

Office européen des brevets

(11) **EP 3 936 097 B1**

(12) **EUROPEAN PATENT SPECIFICATION**

(45) Date of publication and mention
of the grant of the patent:
**04.03.2026 Bulletin 2026/10**

(21) Application number: **20184280.4**

(22) Date of filing: **06.07.2020**

(51) International Patent Classification (IPC):
*A61F 13/15* (2006.01)  *A61F 13/532* (2006.01)
*A61F 13/534* (2006.01)  *A61F 13/539* (2006.01)
*B05C 19/04* (2006.01)

(52) Cooperative Patent Classification (CPC):
**A61F 13/15634; A61F 13/15658; A61F 13/5323;
A61F 13/534; A61F 13/539;** A61F 2013/53463;
A61F 2013/53472; A61F 2013/53908

(54) **APPARATUS AND METHOD FOR THE PRODUCTION OF ABSORBENT ARTICLES WITH IMPROVED CORE**

VORRICHTUNG UND VERFAHREN ZUR HERSTELLUNG VON ABSORBIERENDEN ARTIKELN MIT VERBESSERTEM KERN

APPAREIL ET PROCÉDÉ POUR LA PRODUCTION D'ARTICLES ABSORBANTS DOTÉS D'UNE ÂME AMÉLIORÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.01.2022 Bulletin 2022/02**

(73) Proprietors:
 • **Ontex BV**
  **9255 Buggenhout (BE)**
 • **Ontex Group NV**
  **9320 Erembodegem (BE)**

(72) Inventors:
 • **Garaj, Norbert**
  **56727 Sankt Johann (DE)**
 • **Lambertz, Christina**
  **56566 Neuwied (DE)**
 • **Mailinger, Christel**
  **65604 Elz (DE)**

 • **Ingenfeld, Björn**
  **53115 Bonn (DE)**
 • **Pannwitt, Stefanie**
  **56727 Mayen (DE)**
 • **Heege, Thomas**
  **56761 Düngenheim (DE)**
 • **Weber, Ainas**
  **53474 Bad Neuenahr-Ahrweiler (DE)**
 • **Granado, Martín**
  **56727 Mayen (DE)**

(74) Representative: **Saurat, Thibault**
**Ontex BV**
**Legal Department**
**Korte Keppestraat 21**
**9320 Erembodegem (BE)**

(56) References cited:
EP-A1- 2 905 001      WO-A1-2018/172860
US-A1- 2008 312 618   US-A1- 2016 045 379
US-A1- 2019 159 946

## Description

## TECHNICAL FIELD

The disclosure relates to absorbent articles such as disposable absorbent articles, preferably selected from the group consisting of diapers (whether for baby or adults), pants (whether for baby or adults), pantiliners, briefs, sanitary napkins, and combinations thereof.

## BACKGROUND

Absorbent articles comprising different channel structure designs for enhancing liquid distribution and maximising the use of the core have been developed.

WO2012/170778 (Rosati et al., see also WO2012/170779, WO2012/170781 and WO2012/1708008) discloses absorbent structures that comprise superabsorbent polymer, optionally a cellulosic material, and at least a pair of substantially longitudinally extending channels. The core wrap can be adhesively bonded through the channels to form a channel bond. The channel bonds may be permanent, so that their integrity is at least partially maintained both in dry and wet state. As the absorbent structure absorbs liquid and swells, the absorbent structure takes a three-dimensional shape with the channels becoming visible. The channels provide improved fit and/or liquid acquisition/-transportation, and/or improved performance throughout the use of the absorbent structure.

Further improvements in channel geometries for better core utilisation and liquid distribution are described in EP3342386 describing an absorbent core comprising substantially continuous zones of one or more high fluid distribution structures and discontinuous zones of fluid absorption structures surrounding the one or more high fluid distribution structures, wherein the one or more high fluid distribution structures are arranged to distribute fluid across the absorbent core at a speed that is faster than the speed of fluid distribution across the absorbent core by said discontinuous fluid absorption structures, and wherein said continuous zones extend along a path that is substantially parallel to at least a portion of the perimeter of the core, said portion of the perimeter of the core comprising at least a portion of the sides of the core and one of the ends of the core.

Investment in improved processes for providing channelled absorbent articles has been made. For example WO2018/172860 describes a method for forming an absorbent pad comprising a first layer, a second layer and an absorbent material interposed between the first and the second layer and arranged according to a spreading pattern M1 having at least one channel which is free of absorbent material, comprises a step of feeding a first web (NW1), intended to form the first layer of the pad; a step of feeding a second web (NW2), intended to form the second layer of the pad; a step of spreading the absorbent material on the first web (NW1) according to the spreading pattern M1; a step of joining the first and second webs (NW1, NW2), a step of removing any absorbent material that may be present in the channel.

Another example is EP3453368 that describes a method for manufacturing an absorbent article, said method comprising: a. applying a first binder in a first area on a first side of first sheet material; b. applying a second binder in a second area on a first side of second sheet material; c. applying an absorbent material on the first side of the first sheet material; d. attaching the first sheet material to the second sheet material with the first sides facing each other, such that at least one attachment zone is formed; wherein one of the first sheet material and the second sheet material is a top core wrap sheet material and the other is a back core wrap sheet material; and the first area is arranged at a distance from the intended position of the at least one attachment zone, wherein the first area and the second area are substantially complementary after the step of attaching the wrap sheets to each other.

Documents US2016/0045379 A1, WO2018/172860 A1 and US2008/0312618 A1 also describe methods for manufacturing absorbent articles.

Typically absorbent cores comprise absorbent material that is freely distributed within the core wrap that encloses such absorbent material therein. It has been observed that, due to absence of immobilisation means, the material is prone to collapse during the stress induced by the movement of the wearer. Although core integrity is generally improved for channelled products as some barrier for movement of the absorbent material is created, there is still a need for significant use of adhesive and/or mechanical bonding to achieve reasonable core integrity. It has been further found that degree of immobilization increases the more that absorbent material is maintained (or spatially constricted/restricted) into compartments. In particular, the inventors have found that spatial constriction in the thickness direction is particularly beneficial to restricting movement of the absorbent material, especially when the absorbent material comprises a mixture of cellulose fibers (i.e. fluff pulp) and superabsorbent polymer particles (i.e. SAP).

There is therefore a need for equipment and processes (and absorbent articles) that can improve core stability whilst yet being cost effective and provide cores enhanced liquid handling properties.

## SUMMARY

In a first aspect, the invention relates to an apparatus for manufacturing an absorbent article, said apparatus comprising a supporting member for supporting a first sheet material along a surface thereof, wherein the surface of said supporting member is provided with at least one suction zone and at least one non-suction zone; a first application unit configured for applying a first absorbent material via an airstream on said first sheet material on the supporting member; said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion; a first sheet feed unit configured for applying a second sheet material on top of the first absorbent material on the first sheet material; optionally a first attachment unit configured for attaching said first sheet material to said second sheet material at least in the at least one first attachment portion; a second application unit configured for applying a second absorbent material via an airstream on said second sheet material and/or first absorbent material; said second absorbent material being applied such that said second absorbent material is located on a portion of the second sheet material, and wherein substantially no absorbent material is present on other one or more portions of the second sheet material on at least one second attachment portion; a second sheet feed unit configured for applying a third sheet material on top of the second absorbent material on the second sheet material; a second attachment unit configured for attaching said second sheet material to said third sheet material and/or said first sheet material to said second sheet material at least in the at least one second attachment portion; wherein the first attachment portion and the second attachment portion are substantially congruent or substantially complementary.

In a second aspect, the invention relates to a method for manufacturing an absorbent article, said method comprising the steps of: guiding a first sheet material along a supporting member, wherein a surface of said supporting member is provided with a pattern with at least one suction zone and at least one non-suction zone; applying a first absorbent material via an airstream on said first sheet material on the supporting member, said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion; applying a second sheet material on top of the first absorbent material on the first sheet material; wherein one of said first and second sheet material is a bottom layer of a core wrap substrate, and the other one is an intermediate layer; joining said first sheet material to said second sheet material at least in the at least one first attachment portion, and such that at least one attachment zone is formed; applying a second absorbent material via an airstream on said second sheet material and/or first absorbent material said second absorbent material being applied such that said second absorbent material is located on a portion of the second sheet material, and wherein substantially no absorbent material is present on other one or more portions of the second sheet material on at least one second attachment portion; applying a third sheet material on top of the second absorbent material on the second sheet material; wherein one of said second and third sheet material is an intermediate layer, and the other one is a top layer of a core wrap substrate; joining said second sheet material to said third sheet material at least in the at least one second attachment portion, and such that at least one attachment zone is formed; wherein the first attachment portion and the second attachment portion are substantially congruent or substantially complementary.

## BRIEF DESCRIPTION OF THE FIGURES

**Fig. 1A-D** Illustrates absorbent articles according to embodiments according to the present disclosure.

**Fig. 2A-C** Illustrates absorbent articles according to embodiments according to the present disclosure.

**Fig. 3A-C** Illustrates absorbent articles according to embodiments according to the present disclosure.

**Fig. 4A-C** Illustrates absorbent articles according to embodiments according to the present disclosure.

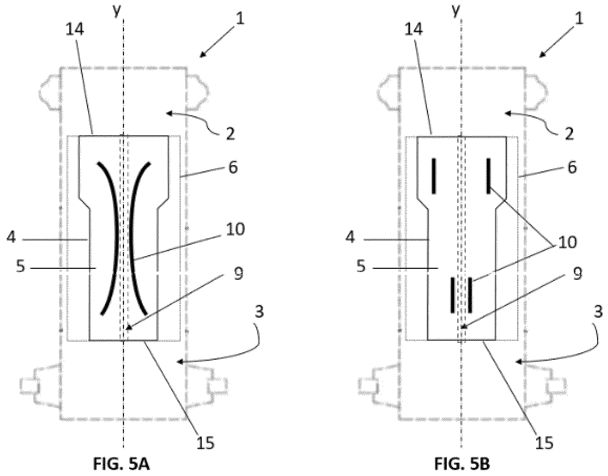

**Fig. 5A-B** Illustrates absorbent articles according to embodiments according to the present disclosure.

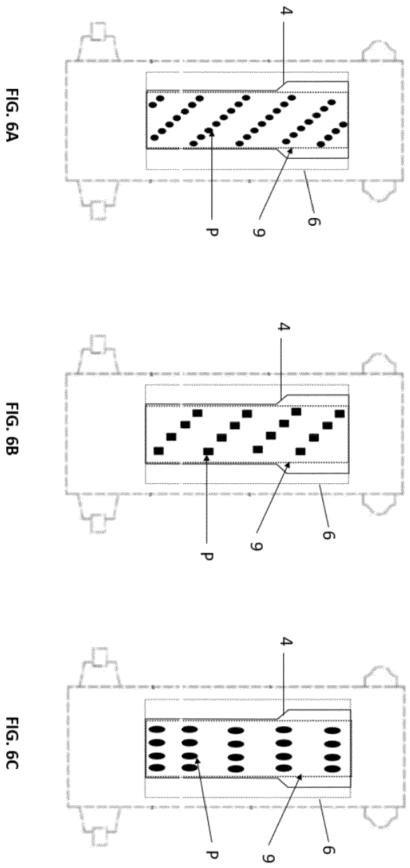

**Fig. 6A-C** Illustrates absorbent articles according to embodiments according to the present disclosure.

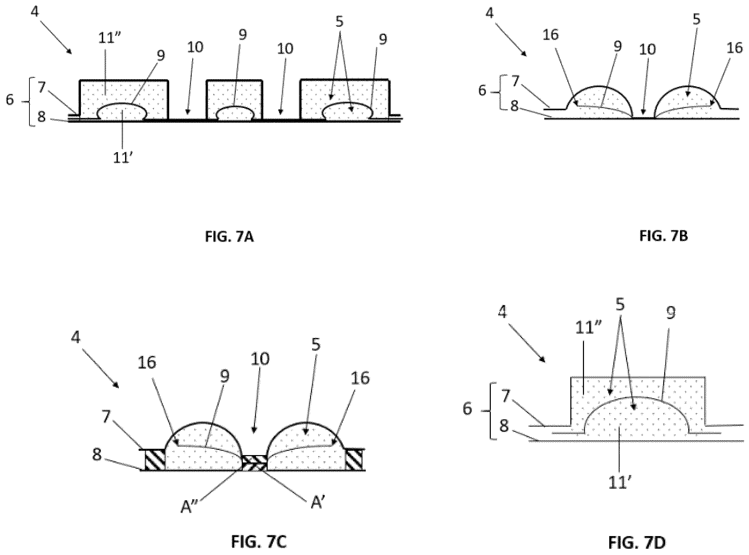

**Fig. 7A-D** schematically illustrate a cross-section of absorbent cores used in absorbent articles according to embodiments of the present disclosure.

Fig. 8 schematically illustrates a cross-section of absorbent cores used in absorbent articles according to embodiments of the present disclosure and showing different bonding positions b.

**Fig. 9A-D** schematically illustrate adhesive patterns according to embodiments of the present disclosure.

Fig. 10 schematically illustrates an apparatus for use in methods of making articles, according to an embodiment of the present disclosure.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements, such as a laminate.

"Laminate" refers to elements being attached together in a layered arrangement.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be preformed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

The recitation of numerical ranges by endpoints includes all numbers and fractions subsumed within that range, as well as the recited endpoints unless otherwise stated.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Adhesive" typically means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, the term "transverse" or "lateral" refers to a line, axis, or direction which lies within the plane of the absorbent article and is generally perpendicular to the longitudinal direction.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 $\mu$m, preferably between 300 to 600 $\mu$m, more preferably between

400 to 500 µm. Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

The expression "channels" as used herein typically refers to structures that form ducts in the absorbent article adapted to conduct liquid therethrough and generally having an aspect ratio (length/width) of greater than 3, typically from 4 to 35. Such channels may be formed by joining an upper layer of the core wrap to a bottom layer of the core wrap such to form channels substantially free of absorbent material flanked by absorbent material, and typically formed on a central region of the absorbent core inboard of the longitudinal and/or transversal edges forming the perimeter thereof. The length is typically the longest dimension and the width is a dimension perpendicular thereto (in the same plane) typically being the shorter dimension. When the structures are non-linear the length is taken as the linear distance between two points or extremities of the structure typically along said longest dimension, and preferably similarly for the width along said shorter dimension.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

"Spunlaced" as used herein refers to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

"Wetlaid" as used herein means nonwovens obtained by a process similar to paper manufacturing. The difference lies in the amount of synthetic fibres present in a wetlaid nonwoven. A dilute slurry of water and fibres is deposited on a moving wire screen, where the water is drained and the fibres form a web. The web is further dewatered by pressing between rollers and dried. Impregnation with binders is often included in a later stage of the process.

"Airlaid" as used herein means a process wherein fibres, which are typcially relatively short, are fed into a forming head by an airstream. The forming head assures a homogeneous mix of all fibres. By air again, a controlled part of the fibre mix leaves the forming head and is deposited on a moving belt, where a randomly oriented web is formed. Compared with carded webs, airlaid webs have a lower density, a greater softness and an absence of laminar structure.

Embodiments of the articles and processes according to the disclosure will now be described. It is understood that technical features described in one or more embodiments maybe combined with one or more other embodiments without departing from the intention of the disclosure and without generalization therefrom.

## THE ABSORBENT ARTICLE

In an embodiment, absorbent article (1) comprises: a liquid permeable topsheet (2), a liquid impermeable backsheet (3), and an absorbent core (4) positioned between said topsheet (2) and backsheet (3), wherein the absorbent core (4) comprises an absorbent material (5), said absorbent material comprising cellulose fibers and/or superabsorbent polymers, and wherein said absorbent material is contained within at least one core wrap substrate (6) enclosing said absorbent material therein, wherein the absorbent core further comprises an intermediate layer (9) positioned between a top layer (7) of said core wrap and a bottom layer (8) of said core wrap such that said absorbent material (5) is disposed between said top layer (7) and said intermediate layer (9) and/or between said bottom layer (8) and said intermediate layer (9), and wherein said top layer (7), said bottom layer (8), and said intermediate layer (9) are joined together to form one or more channel forming areas (10) substantially free of said absorbent material. Advantageously, this arrangement allows to improve core integrity in an effective way and at low cost.

Preferably, at least the absorbent material comprised between said intermediate layer (9) and said bottom layer (8) comprises a mixture of cellulose fibers and superabsorbent polymer particles (typically comprising more than 10%wt, preferably from 15% to 30%, of cellulose fibers by weight of the absorbent material).

Preferably, the absorbent material (5) comprises a mixture of superabsorbent polymer particles and cellulose fibers (preferably at the amounts described hereinbelow) at least in one, preferably both, of: (i) between said top layer (7) and intermediate layer (9); and (ii) between said intermediate layer (9) and said bottom layer (8). Most preferably wherein said mixture comprises more cellulose fibers between said intermediate layer (9) and said bottom layer (8) than the amount of cellulose fibers comprised between said top layer (7) and intermediate layer (9). Advantageously this allows to provide better rewet performance and provide an improved dryness perception upon wetting.

In an embodiment, the absorbent material comprises first superabsorbent polymer particles between said top layer (7) and intermediate layer (9); and second superabsorbent polymer particles between said intermediate layer (9) and said bottom layer (8); wherein said first and second superabsorbent polymer particles have different properties, said properties selected from the group consisting of vortex (seconds); absorbency under load (AUL) at 0.7 psi (g/g); and combinations thereof. Preferably wherein the second superabsorbent polymers have a lower absorbtion speed (herein also referred to as vortex) and/or absorbency under load than said first superabsorbent polymers. More preferably wherein: (a) the first absorbent polymer particles have an AUL of more than 18 g/g, preferably from 20 g/g to 50 g/g; and/or a vortex of more than 50s, preferably from 60s to 90s; and/or (b) the second absorbent polymer particles have an AUL of less than 18 g/g, preferably from 5 g/g to 15 g/g; and/or a vortex of less than 50s, preferably from 15s to 45s. Advantageously this allows for improved liquid absorption performance.

In a preferred embodiment, at least the second superabsorbent polymer particles comprise biocompatible and/or biodegradable superabsorbent polymer particles such as clay, gelatin, and/or sugar comprising particles. In an embodiment, the biocompatible, biodegradable, macromolecular water-absorbent hybrid material (WAHM), has a three-dimensional configuration with intermolecular covalent bonds and containing free functional groups selected from OH, SH, NH2 and COOH, said polymer being formed by polymer-polymer intercoupling reaction between a natural water-soluble polymer A or its derivatives having a molecular weight between 20,000 and 300,000 Da, and a synthetic polymer B in an adequate ratio between 1 and 50% from dried mixture (A+B), wherein the natural polymer A is selected from: amphoteric reactants, partially denatured or chemically modified natural polymer, that dissociates in water to form both anions and cations, and which can undergo polymer-polymer intercoupling reactions, and wherein: synthetic polymer B is a linear or branched reactive synthetic copolymer having a molecular weight of 50,000-500,000 Da derived from a vinyl monomer and an ethylenically unsaturated monomer, said copolymer having a backbone with polymeric subunits Rn and Rf, wherein R represents a subunit covalently bonded to the polymer backbone, n represents nonreactive chemical functional groups and f represents reactive chemical functional groups, as generally exemplified in US20090306290, in particular examples 1 to 8. Indeed without wishing to be bound by theory, such SAPs although beneficial for the environment show higher rewet behaviour, it is thus desirable to include such materials below the intermediate layer (and above the bottom layer (8)) in order to ensure improved dryness on the skin surface.

The cores described herein may comprise more than one intermediate layer (9). Preferably wherein each intermediate layer (9) comprises absorbent material on both a garment-facing surface and body-facing surfaces thereof. Tyically wherein all intermediate layers (9) are contained and/or sandwiched between the top layer (7) and bottom layer (8). Advantageously, a multilayer core can be made which can further compartmentalise the absorbent material along the thickness direction and hence providing core stability as well as modulating liquid flow through the core with different SAP/fluff concentrations and SAP grades.

When a plurality of intermediate layers (9) are comprised, it is preferable that at least two of said layers (9) have a different width (generally taken along the transversal axis that runs perpendicular to the longitudinal axis y), preferably wherein said width is less than the width of the top and/or bottom layers (7, 8). Preferably, the intermediate layer being closest to the top layer (7) having the greatest or smallest, preferably the smallest, width compared to other intermediate layers. This arrangement allows for a multi-layer stacked core that however limits stiffening the core structure especially upon wetting thus improving the overall comfort as well as providing absorbancy benefits. Moreover, having an intermediate layer closest to the top layer being smallest in width ensures reduced risk of rewet on the topsheet side of the article.

When the core comprises a plurality of intermediate layers (9), the width thereof of each said intermediate layers is typically less than that of the top and bottom layers (7, 8) such that the top and bottom layers may be joined to each other at least along a portion of the perimeter thereof to sandwich said intermediate layers (and absorbent material) therebetween. The plurality of intermediate layers (9) may consist of the same nonwoven having different basis weight in $g/m^2$, or may consist of different materials such as different nonwovens or films as described herein below.

In a preferred embodiment, the basis weight of the intermediate layer (9) is greater or equal to the basis weight of the top and/or bottom layers (7, 8). Typically the top and/or bottom layers (7, 8) have a basis weight of from 5 gsm to 45 gsm, preferably 7 gsm to 40 gsm, more preferably 8 gsm to 35 gsm, even more preferably from 9 gsm to 30 gsm, even more preferably from 10 gsm to 28 gsm.

It is highly preferred that the intermediate layer (9) has a basis weight of greater than 5 gsm, preferably greater than 10 gsm ($g/m^2$) and less than 80 gsm ($g/m^2$), preferably from 15 gsm to 75 gsm, more preferably from 20 gsm to 70 gsm, more preferably from 25 gsm to 65 gsm, more preferably from 30 gsm to 60 gsm, more preferably from 35 gsm to 55 gsm, even more preferably from 40 gsm to 50 gsm. Advantageously this arrangement allows for good mechanical integrity that supports overall core integrity whilst enhancing liquid distribution whilst eliminating unnecessary cost of higher basis weights. Moreover, increasing the basis weight more that the described upper limits leads to, during use, an increase risk of inadvertent release of the bonds joining the top/bottom core wrap layers to the intermediate layer due to an increase risk of absorbent material (especially fluff) contamination that weakens the bonding strength.

In an embodiment, the intermediate layer (9) is joined, preferably directly, to the top layer (7) and the bottom layer (8) of the core wrap substrate (6) such that said top layer (7) is joined to a body-facing surface of the intermediate layer (9) and the bottom layer (8) is joined to a garment-facing surface of the intermediate layer (9), typically at least within the channel forming areas (10).

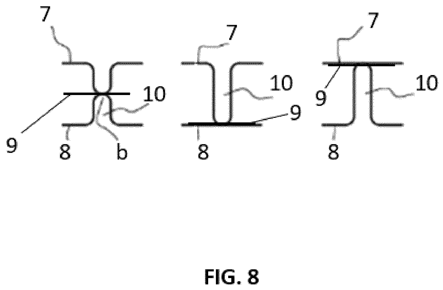

In an embodiment, as illustrated in Fig. 8, the top, intermediate, and bottom layers (7, 9, 8) are joined together in the channel forming areas (10) at a bonding position (b) that may be substantially in the middle, bottom or top of a thickness of the absorbent core.

Preferably, the absorbent core (4) comprises a plurality of clusters (11) of absorbent material (5) wherein more than one, preferably the majority, even more preferably each, of said clusters is circumscribed by said channel forming areas (10), and wherein each said cluster is separated from the neighboring cluster by said channel forming areas (10). Advantageously such clusters form defined compartments for absorbent material that is retained and prevented from dislodging during use especially when wetted.

In an embodiment, the top layer (7), the bottom layer (8), and the intermediate layer (9) are joined together by one or more adhesives. Alternatively or in addition, the top layer (7), the bottom layer (8), and the intermediate layer (9) are joined together by one or more mechanical bonds selected from the group consisting of ultrasonic bonds, thermal bonds, pressure bonds, and combinations thereof. When adhesive and mechanical bonds are combined, they are preferably applied in different portions of the channel forming areas (10) so that no same portion comprises both adhesive and mechanical bond. Advantageously this allows to modulate the stiffness and degree of wet integrity of the clusters that may be thus arranged to release upon swelling when wetted in some regions of the absorbent core.

In an embodiment, the channel forming areas (10) are interconnected such that they extend both along a longitudinal axis (y) and a transversal axis, running substantially perpendicular to the longitudinal axis (y). This allows liquid to flow through the channels along both the longitudinal direction and transverse direction of the absorbent core and be absorbed by neighboring clusters as it makes its way along the channel, hence making more effective use of the core via proper liquid distribution across its entire surface.

In an embodiment, the absorbent core (4) comprises a first terminal edge cluster (12) and/or a second terminal edge cluster (13) positioned on opposite front and/or back transverse edges (14, 15) of the core respectively (generally respectively positioned on the front (F) and/or back (B) of the absorbent article), and arranged such that channel(s) formed by the channel forming areas (10) are separated from said front and/or back transverse edges (14, 15) by said first terminal edge cluster (12) and/or said second terminal edge cluster (13) respectively. Advantageously, these behave like barriers to leakage by ensuring a large space of absorbent material to be present along such terminal edges so that liquid is quickly absorbed before reaching the edge and hence reducing the likelihood of leakage.

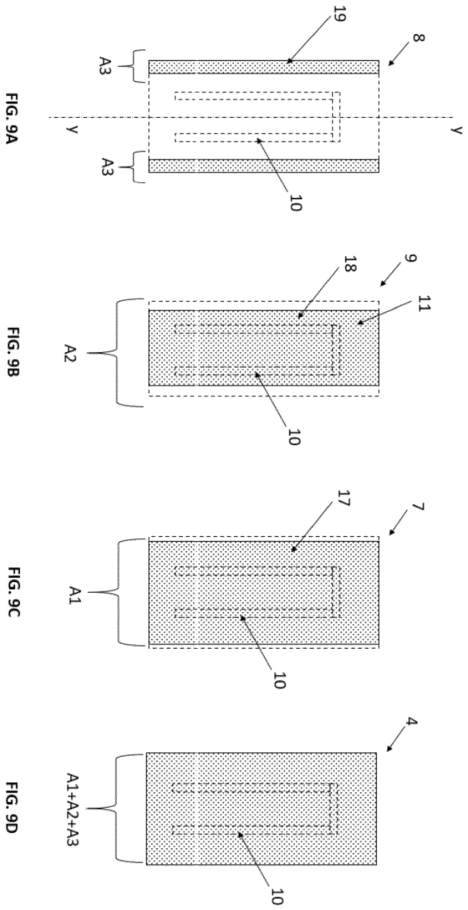

In an embodiment, as illustrated in Fig. 9A-D, the top layer (7) comprises a first adhesive (17) arranged to define a first adhesive area (A1), the intermediate layer (9) comprises a second adhesive (18) arranged to define a second adhesive area (A2), and the bottom layer (8) comprises a third adhesive (19) arranged to define a third adhesive area (A3), wherein said first adhesive area (A1) and the second adhesive area (A2) are greater than the third adhesive area (A3), and preferably wherein said first, second, and third adhesive areas (A1, A2, A3) comprise one or more adhesive stripes. Preferably, wherein the first adhesive layer (A1) is greater or equal to the second adhesive area (A2), and preferably at least one of said adhesive stripes of the first adhesive area (A1) and the second adhesive area (A2) overlaps said channel forming areas (10), and wherein at least one, preferably at least two, of said adhesive stripes of the third adhesive area (A3) is positioned outboard of said channel forming areas (10). Preferably, wherein the third adhesive area (A3) consists of a plurality of spaced apart adhesive stripes wherein at least one, preferably at least two, more preferably at least three, even more preferably from 4 to 7, of said adhesive stripes is positioned outboard and/or inboard of the channel forming areas (10). Advantageously this allows for optimal attachment of the layers in the channel forming areas whilst limiting contamination (e.g. absorbent material sticking into the channel regions prior to joining the other layers in said channel forming areas).

In an embodiment, the first and/or second adhesive areas (A1, A2) comprise a substantially uniform single stripe of adhesive arranged to overlap at least a substantial portion of the channel forming areas (10).

Preferably, the intermediate layer (9) is selected from the group consisting of a nonwoven, film, and combinations thereof, preferably a nonwoven selected from the group consisting of wetlaid, airlaid, carded, spunbond, meltblown, carded thermobonded, air-through-bonded, spunlaced, tissue, and combinations thereof, more preferably a nonwoven selected from the group consisting of carded thermobonded, air-through-bonded, spunlaced, and combinations thereof, most preferably a nonwoven selected from the group consisting of air-through-bonded, spunlaced, and combinations thereof, most preferably a spunlaced nonwoven. Such nonwovens can comprise synthetic or natural fibers. Synthetic fibers are typically selected from the group consisting of polyethylene (PE), polypropylene (PP), polylactic acid (PLA), and mixtures thereof. Natural fibers are typically cellulosic and may be selected from the group consisting of cotton, rayon, micro-fibril cellulose (MFC), derivatives thereof, and mixtures thereof. Most preferred are spunlaced nonwovens generally comprising natural fibers. A particular advantage of spunlaced nonwovens is not only its environmentally friendly impact but further this material normally soaks up liquid and thus is generally seen in the industry as undesirable in view of the rewet disadvantages in absence of other technologies to limit this drawback, in this case however this is turned into an advantage as the layer behaves as a liquid distribution layer within the core itself and thus promoting further distributed absorption by the absorbent material being present both above and below such layer.

In an embodiment, the top layer (7) and bottom layer (7) are the same or different, preferably different, from the intermediate layer (9), and are preferably a nonwoven selected from the group consisting of spunbond, meltblown, carded thermobonded, and combinations thereof.

In an embodiment, the intermediate layer (9) has a width, extending along an axis substantially perpendicular to a longitudinal axis (y), that is less than or equal to a width, extending along an axis substantially perpendicular to a longitudinal axis (y), of the top and/or bottom layers (7,8) of the core wrap. Especially when the width is less than that of the core wrap core stability is retained whilst limiting cost and use of raw material.

Preferably, the intermediate layer comprises at least one, preferably two, free end (16) that is not joined to the top layer (7) and/or bottom layer (8), preferably two oppositely disposed free ends such that each free end is substantially surrounded by absorbent material, preferably said absorbent material being disposed on a garment facing surface, skin facing surface, and lateral edge, of each free end. Advantageously, this allows the intermediate layer to act not only as core integrity system but also further as a liquid distribution layer with the free ends being free to transport liquid directly in middle of the absorbent material and increasing the surface area in contact thereto for optimal liquid transport.

**In an embodiment,** the top layer (7) is, preferably directly, joined to the bottom layer (8) at one or more positions being outboard of the intermediate layer (9) and typically at the lateral or longitudinal edges forming the perimeter of the absorbent core and extending substantially parallel to a longitudinal axis (y). Said edges may also be folded over themselves to form a C-fold (typically such that folded flaps are in contact twith the body-facing surface of the top layer (7) or the garment-facing surface of the bottom layer (8) and can be held in position via adhesive and/or mechanical bonding). Advantageously, this allows to reduce the risk of absorbent material leaking out of the core during use.

In an embodiment, the intermediate layer is bonded substantially only in the channel forming areas (10), and typically not along the lateral or longitudinal edges forming the perimeter of the absorbent core and extending substantially parallel to a longitudinal axis (y).

In another embodiment, the absorbent article (1) comprises: a liquid permeable topsheet (2), a liquid impermeable backsheet (3), and an absorbent core (4) positioned between said topsheet (2) and backsheet (3), wherein the absorbent core (4) comprises an absorbent material (5), said absorbent material comprising cellulose fibers and/or superabsorbent polymers, and wherein said absorbent material is contained within at least one core wrap substrate (6) enclosing said absorbent material therein, wherein the absorbent core further comprises an intermediate layer (9) positioned between a top layer (7) of said core wrap and a bottom layer (8) of said core wrap such that said absorbent material (5) is disposed between said top layer (7) and said intermediate layer (9) and between said bottom layer (8) and said intermediate layer (9). Advantageously this allows to achieve better core integrity, especially in the thickness direction in an effective and cheaper way.

In an embodiment, said top layer (7), said bottom layer (8), and said intermediate layer (9) are joined together at one or more distinct positions arranged such that said absorbent material (5) is present over substantially the entirety of the core.

Preferably, the absorbent core (4) is free of channels substantially free of absorbent material. This arrangement allows to maintain a larger surface area of the core being covered by absorbent material of effective high absorbency, whilst retaining the advantages in core integrity that are normally provided by channels.

In an embodiment, the absorbent core (4) comprises a perimeter having first and second transverse edges (14, 15) and first and second longitudinal edges (14',15') connecting said transverse edges (14,15) and extending parallel to a longitudinal axis (y), and wherein the intermediate layer (9) is joined, preferably directly, to the top layer (7) and the bottom layer (8) of the core wrap substrate (6) such that said top layer (7) is joined to a body-facing surface of the intermediate layer (9) and the bottom layer (8) is joined to a garment-facing surface of the intermediate layer (9), preferably only, along at least one of the edges (14,14',15,15') of the perimeter of the absorbent core (4). This allows to avoid bonding positions within the core central area whilst at the same time retaining core integrity benefits.

In an embodiment, the absorbent core (4) comprises no more than two compartments or clusters of absorbent material, and typically at most a top compartment or cluster (11') between the top layer (7) and the intermediate layer (9); and a bottom compartment or cluster (11") between the bottom layer (8) and the intermediate layer (9).

In an embodiment, the top layer (7), the bottom layer (8), and the intermediate layer (9) are joined together by one or more adhesives. Alternatively or in addition, the top layer (7), the bottom layer (8), and the intermediate layer (9) may be joined together by one or more mechanical bonds selected from the group consisting of ultrasonic bonds, thermal bonds, pressure bonds, and combinations thereof.

In an embodiment, the intermediate layer (9) is joined to the top layer (7) and/or the bottom layer (8) at one or more bonding points (P) positioned inboard of the perimeter of the absorbent core (4), and preferably wherein said bonding points have an aspect ratio of less than 3, preferably of from 1 to 2, and more preferably having a shape selected from the group consisting of circular, elliptical, line-form, star-shaped, polygonal, and combinations thereof. Advantageously this results in an absorbent core that not only significantly increases core integrity but further promotes liquid distribution and core usage in absence of channels that normally may result in greater sagging when wetted, hence also improving overall fit when worn.

Preferably, the cores herein comprise absorbent material comprising, preferably consisting of, a mixture of superabsorbent polymer particles (SAP) and cellulose fibers (or fluff pulp), typically arranged such that each cluster or compartment comprises said mixture. More preferably the absorbent material comprises less than 35%wt, preferably from 5%wt to 30%wt, more preferably from 11%wt to 25%wt of cellulose fibers; and more than 60%wt, preferably more than 65%wt, even more preferably from 70%wt to 95%wt, even more preferably from 75%wt to 85%wt, of SAP; by weight of the absorbent material. Advantageously it has been found that unlike fluffless cores (i.e. free of cellulose fibers), such clustered arrangements comprising a mixture with fluff but at the above specified low ranges provides for added speed of liquid distribution and uptake by the SAP whilst still enjoying the benefits of having a considerably thinner core compared to fluff-containing cores of the prior art, this especially without the need for investing in special and costly SAP polymers that would be needed to match said distribution and uptake properties normally otherwise resulting by added gelblocking effects.

THE APPARATUS

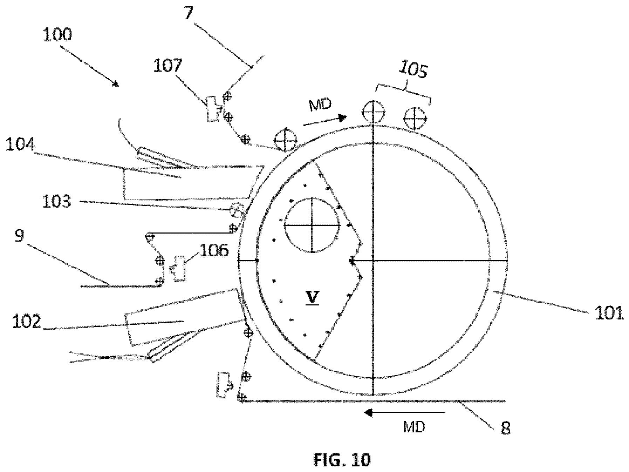

According to an aspect of the invention and as exemplified in Fig. 10, an apparatus (100) for manufacturing an absorbent article comprises: a supporting member (101) for supporting a first sheet material (8) along a surface thereof, wherein the surface of said supporting member (101) is provided with at least one suction zone and at least one non-suction zone; a first application unit (102) configured for applying a first absorbent material by depositing absorbent material via an airstream, e.g. blowing on said first sheet material (8) (herein interchangeably referred to as bottom layer) on the supporting member (101); said first absorbent material being applied such that said first absorbent material is located on a portion of the first sheet material (8) corresponding to the at least one suction zone, and wherein substantially no absorbent material is present on other one or more portions of the first sheet material (8) corresponding to the at least one non-suction zone on at least one first attachment portion (A'); a first sheet feed unit configured for applying a second sheet material (9) (herein interchangeably referred to as intermediate layer) on top of the first absorbent material on the first sheet material (8); optionally a first attachment unit (103) configured for attaching said first sheet material (8) to said second sheet material (9) at least in the at least one first attachment portion (A'); a second application unit (104) configured for applying a second absorbent material by depositing absorbent material via an airstream, e.g. blowing on said second sheet material (9) and/or first absorbent material; said second absorbent material being applied such that said second absorbent material is located on a portion of the second sheet material (9), and wherein substantially no absorbent material is present on other one or more portions of the second sheet material (9) on at least one second attachment portion (A"); a second sheet feed unit configured for applying a third sheet material (7) (herein interchangeably referred to as top layer) on top of the second absorbent material on the second sheet material (9); a second attachment unit (105) configured for attaching said second sheet material (9) to said third sheet material (7) and/or said first sheet material (8) to said second sheet material (9) at least in the at least one second attachment portion (A"); wherein the first attachment portion (A') and the second attachment portion (A") are substantially congruent or substantially complementary typically in a planar view (i.e. plane formed by a length and width of the absorbent core typically corresponding to the longitudinal axis (y) and the transverse axis perpendicular thereto).

In a preferred embodiment, at least the first application unit (102) (but preferably all the application units described herein) comprises a blowing means (such as an air stream source) to direct the absorbent material onto the bottom layer/first sheet material (8) and/or the intermediate layer/second sheet material (9), and is preferably a non-contact application unit typically meaning or in that it is free of a rotatable laying-out roller comprising a plurality of pockets for applying a spread of absorbent material (also conventionally known or referred to as absorbent material printing). Especially when the absorbent material comprises cellulose fibers it is desirable to apply such absorbent material via a non-contact application in order to achieve good mixing and spacing apart of the superabsorbent polymer particles between cellulose fibers and limit agglomeration of said particles, this allowing to achieve cores with better liquid distribution properties along and across its surface.

In an embodiment, the supporting member (101) is a rotating drum and the at least one non-suction zone comprises at least one elongate zone extending in a circumferential direction of the rotating drum.

In an embodiment, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In an embodiment, the apparatus further comprises a removing unit comprising a mechanical removal means configured for locally removing the absorbent material applied on said first and/or second sheet material above the at least one non-suction zone.

Preferably, wherein the mechanical removal means comprises one or more roller brush or air blower.

In an embodiment, the first sheet feed unit is positioned between the first application unit (102) and the second application unit (104), and typically upstream of the first attachment unit (103) along a machine direction (MD). Advantageously this allows to ensure reduced risk of contamination between layers.

In an embodiment, the apparatus further comprises a first adhesive unit (106) arranged to apply an adhesive pattern on a surface of the second sheet material (9) facing the first absorbent material; and a second adhesive unit (107) arranged to apply an adhesive pattern on a surface of the third sheet material (7) facing the second absorbent material; said first and second adhesive units being positioned upstream of said first and second attachment units respectively.

In an embodiment, the first and/or second attachment units (103, 105) comprise a pressure means, such as a pressure roller (typically having a substantially smooth upper surface generally such that it is free of protrusions pressing into the channel forming areas so as to limit the need for process registration), arranged to provide an adhering force to join the first, second, and/or third sheet materials respectively; and preferably wherein the first attachment unit (103) comprises a single pressure means, and the second attachment unit (105) comprises a plurality of pressure means (preferably at least three pressure means arranged sequentially along a machine direction).

In an embodiment, the first attachment unit (103) is positioned between the first application unit (102) and the second application unit (104), and downstream of the first sheet feed unit typically along a machine direction (MD). Advantageously this allows to ensure optimal adhesion and reduced risk of contamination between layers.

In an embodiment, the supporting member (101) is a rotating drum and the at least one non-suction zone comprises at least one elongate zone extending in a circumferential direction of the rotating drum, preferably a plurality of said non-suction zones are comprised in said pocket. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In an embodiment, the apparatus further comprises a removing unit comprising a mechanical removal means configured for locally removing the absorbent material applied on said first and/or second sheet material above the at least one non-suction zone. Typically, wherein the mechanical removal means comprises one or more roller brush or air blower.

In an embodiment, the apparatus further comprises a first adhesive unit (106) arranged to apply an adhesive pattern on a surface of the second sheet material facing the first absorbent material; and a second adhesive unit (107) arranged to apply an adhesive pattern on a surface of the third sheet material facing the second absorbent material; said first and second adhesive units being positioned upstream of said first and second attachment units (103, 105) respectively. The apparatus may further comprise a further adhesive unit arranged to apply an adhesive pattern on a surface of the first sheet material facing the first absorbent material.

In an embodiment, the first and second attachment units comprise a pressure means, such as a pressure roller (typically having a substantially smooth contact surface generally such that it is free of protrusions pressing into the channel forming areas so as to limit the need for process registration), arranged to provide an adhering force to join the first, second, and third sheet materials respectively; and preferably wherein the first attachment unit comprises a single pressure means, and the second attachment unit comprises a plurality, preferably from 2 to 5, of pressure means.

In another embodiment, the apparatus (100) used to make absorbent articles herein comprises:

a supporting member (101) for supporting a first sheet material (being the bottom layer (8) along a surface thereof, typically said supporting member (101) comprising a plurality of said pockets generally disposed along a circumference thereof, wherein the surface of said supporting member (101) is provided with at least one suction zone and at least one non-suction zone;
a first application unit (102) configured for applying a first absorbent material (typically by depositing absorbent material via an airstream, e.g. blowing) on said first sheet material on the supporting member (101); said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion (A');
a first sheet feed unit configured for applying a second sheet material (being the intermediate layer (9)) on top of the first absorbent material on the first sheet material; optionally a first attachment unit (103) configured for attaching said first sheet material to said second sheet material at least in the at least one first attachment portion (A');
a second application unit (104) configured for applying a second absorbent material (typically by depositing absorbent material via an airstream, e.g. blowing) on said second sheet material and/or first absorbent material; said second absorbent material being applied such that said second absorbent material is located on a portion of the second sheet material, and wherein substantially no absorbent material is present on other one or more portions of the second sheet material on at least one second attachment portion (A");
a second sheet feed unit configured for applying a third sheet material (being the top layer (7)) on top of the second absorbent material on the second sheet material;
a second attachment unit (105) configured for attaching said second sheet material to said third sheet material (7) at least in the at least one second attachment portion (A"); wherein the first attachment portion (A') and the second attachment portion (A") are substantially congruent or substantially complementary (typically when viewed in a planar view of the absorbent core).

In an embodiment, the supporting member (101) is a rotating drum and the at least one non-suction zone comprises at least one elongate zone extending in a circumferential direction of the rotating drum, preferably a plurality of said non-suction zones are comprised in said pocket. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In another embodiment, the apparatus (100) used to make absorbent articles herein comprises:

a supporting member (101) for supporting a first sheet material (being the bottom layer (8) along a surface thereof, wherein the surface of said supporting member (101) is provided with at least one suction zone and at least one non-suction zone;
a first application unit (102) configured for applying a first absorbent material (typically by depositing absorbent material via an airstream, e.g. blowing) on said first sheet material on the supporting member (101); said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion (A');

a first sheet feed unit configured for applying a second sheet material (being the intermediate layer (9)) on top of the first absorbent material on the first sheet material; optionally a first attachment unit (103) configured for attaching said first sheet material to said second sheet material at least in the at least one first attachment portion (A');

a second application unit (104) configured for applying a second absorbent material (typically by depositing absorbent material via an airstream, e.g. blowing) on said second sheet material and/or first absorbent material; said second absorbent material being applied such that said second absorbent material is located on a portion of the second sheet material, and wherein substantially no absorbent material is present on other one or more portions of the second sheet material on at least one second attachment portion (A");

a second sheet feed unit configured for applying a third sheet material (being the top layer (7)) on top of the second absorbent material on the second sheet material;

a second attachment unit (105) configured for attaching said second sheet material to said third sheet material (7) at least in the at least one second attachment portion (A"); wherein the first attachment portion (A') and the second attachment portion (A") are substantially congruent or substantially complementary (typically when viewed in a planar view of the absorbent core).

In an embodiment, the supporting member (101) is a rotating drum and the at least one non-suction zone comprises at least one elongate zone extending in a circumferential direction of the rotating drum. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

THE PROCESS OF MAKING

According to an aspect of the invention, the method for manufacturing an absorbent article, comprises the, preferably the sequential, steps of: guiding a first sheet material (8) along a supporting member (101), wherein a surface of said supporting member is provided with a pattern with at least one suction zone and at least one non-suction zone; applying a first absorbent material via an airstream, e.g. blowing on said first sheet material (8) on the supporting member, said first absorbent material being applied such that said first absorbent material is located on a portion of the first sheet material (8) corresponding to the at least one suction zone, and wherein substantially no absorbent material is present on other one or more portions of the first sheet material (8) corresponding to the at least one non-suction zone on at least one first attachment portion (A'); applying a second sheet material (herein also referred to as intermediate layer (9)) on top of the first absorbent material on the first sheet material (8); wherein one of said first and second sheet material is a bottom layer of a core wrap substrate (6), and the other one is an intermediate layer; joining said first sheet material (8) to said second sheet material (9) at least in the at least one first attachment portion (A'), and such that at least one attachment zone is formed; applying a second absorbent material via an airstream, e.g. blowing on said second sheet material (9) and/or first absorbent material; said second absorbent material being applied such that said second absorbent material is located on a portion of the second sheet material (9), and wherein substantially no absorbent material is present on other one or more portions of the second sheet material (9) on at least one second attachment portion (A"); applying a third sheet material (7) on top of the second absorbent material on the second sheet material (9); wherein one of said second and third sheet material is an intermediate layer, and the other one is a top layer of a core wrap substrate (6); joining said second sheet material (9) to said third sheet material (7) at least in the at least one second attachment portion (A"), and such that at least one attachment zone is formed; wherein the first attachment portion (A') and the second attachment portion (A") are substantially congruent or substantially complementary typically in a planar view.

Optionally, the method further comprises the step of locally removing the absorbent material applied on said first and/or second sheet material, preferably by a mechanical removal means preferably comprising one or more roller brush or air blower. Typically such to actively decontaminate the bonding regions from absorbent material.

In an embodiment, the method further comprises the step of applying a first adhesive pattern on a surface of the second sheet material (9) facing the first absorbent material; and applying an second adhesive pattern on a surface of the third sheet material (7) facing the second absorbent material, preferably wherein the first and second patterns are substantially the same or different.

Preferably, the joining step(s) (at least the joining of the intermediate layer (9) to the bottom layer (8)) comprises the step of applying a pressure and/or adhering force to join the first, second, and/or third sheet materials respectively, substantially concurrently with a suction force within the at least one suction zone typically such to combine a downward push with a substantially simultaneous downward pull wherein downward is the direction from the position at which pressure is applied to or towards the supporting member. This is typically achieved by ensuring that the attachment unit(s) herein is/are disposed such to superpose a vacuum region (V) within the support unit (typically in the form of a rotating drum). Advantageously this allows to attain good and strong adhesion without having to apply excessive pressures (with the use of e.g. pressure rollers or embossing rollers) that may inadvertently damage sections of the absorbent core.

In a preferred embodiment, the vacuum region (V) extends only over a section of the support unit, and when said support unit is in the form of a rotating drum it extends over an angle of more than 30° to less than 200°, preferably at an angle of from 45° to 180°. Advantageously this allows for ensuring a vacuum force where needed whilst providing energy savings.

Preferably, the pattern comprises a plurality, preferably more than 4, even more preferably from 5 to 20, of spaced apart suction zones, typically in the form of distinct cavities (generally within a same pocket that typically corresponds to one absorbent core of an absorbent article in a series of articles made in substantial sequence), and at least one non-suction zone, such that a plurality of clusters of absorbent material are formed typically after respective application steps.

In another embodiment, the method for making an absorbent article comprises the steps of:

> i. providing a pocket comprising a single porous cavity (as used herein the porous cavity is typically intended as a cavity comprising a base having a plurality of openings to form a porous base), wherein said cavity is in fluid communication with an under-pressure source;
> ii. providing a first nonwoven web in the form of a bottom layer (8) of a core wrap;
> iii. depositing said bottom layer (8) onto said pocket;
> iv. depositing a first absorbent material, comprising cellulose fibers and/or superabsorbent polymer particles, (typically via an airstream, e.g. blowing) over at least a portion of a surface of said bottom layer (8);
> v. depositing an intermediate layer (9) over the first absorbent material such that said first absorbent material is sandwiched between said intermediate layer (9) and bottom layer (8);
> vi. joining said intermediate layer (9) to said bottom layer (8) at one or more first distinct positions;
> vii. depositing a second absorbent material, comprising cellulose fibers and/or superabsorbent polymer particles, (typically via an airstream, e.g. blowing) over at least a portion of a said intermediate layer (9);
> viii. depositing a second nonwoven web in the form of a top layer (7) of a core wrap over the second absorbent material such that said second absorbent material is sandwiched between said intermediate layer (9) and top layer (7);
> ix. joining said intermediate layer (9) to said top layer (7) and/or bottom layer (8) at one or more second distinct positions, to form an absorbent core;
> x. optionally joining an acquisition distribution layer to the body facing surface of said top layer (7), and preferably laminating the absorbent core and the acquisition distribution layer between a liquid pervious topsheet and a liquid impervious backsheet;

wherein the absorbent core (4) comprises no more than two compartments or clusters of absorbent material corresponding to said single cavity, and typically at most a top compartment or cluster between the top layer (7) and the intermediate layer (9); and a bottom compartment or cluster between the bottom layer (8) and the intermediate layer (9).

In an embodiment, the first and second distinct positions are the same or different, and wherein said first and/or second distinct positions comprise one or more bonding points positioned inboard of the perimeter of the absorbent core (4), and preferably wherein said bonding points have an aspect ratio of less than 3, preferably of from 0.5 to 2.5, and more preferably having a shape selected from the group consisting of circular, elliptical, line-form, star-shaped, polygonal, and combinations thereof, and preferably wherein said bonding points comprise mechanical bonds.

In another embodiment, the method for making an absorbent article comprises the, preferably substantially sequential, steps of:

> i. providing a pocket comprising a plurality of porous cavities, wherein said cavities are in fluid communication with an under-pressure source;
> ii. providing a first nonwoven web in the form of a bottom layer (8) of a core wrap;
> iii. optionally applying an adhesive pattern, preferably in the form of a plurality of adhesive stripes, to said bottom layer (8);
> iv. depositing said bottom layer (8) onto said pocket, preferably such that said adhesive pattern faces away from said pocket;
> v. depositing a first absorbent material (typically via an airstream, e.g. blowing), comprising cellulose fibers and/or superabsorbent polymer particles, over at least a portion of a surface of said bottom layer (8);
> vii. optionally applying a second adhesive pattern to an intermediate layer (9);
> viii. depositing an intermediate layer (9) over the first absorbent material such that said first absorbent material is sandwiched between said intermediate layer (9) and bottom layer (8);
> ix. joining said intermediate layer (9) to said bottom layer (8) to form one or more channel forming areas (10) substantially free of absorbent material and a plurality of first clusters (11') of absorbent material corresponding to said porous cavities;
> x. depositing a second absorbent material (typically via an airstream, e.g. blowing), comprising cellulose fibers and/or superabsorbent polymer particles, over at least a portion of a said intermediate layer (9);
> xi. optionally applying a third adhesive pattern to a second nonwoven web in the form of a top layer (7) of a core wrap;

xii. depositing a second nonwoven web in the form of a top layer (7) of a core wrap over the second absorbent material such that said second absorbent material is sandwiched between said intermediate layer (9) and top layer (7);

xiii. joining said intermediate layer (9) to said top layer (7) to form one or more channel forming areas (10) substantially free of absorbent material and a plurality of second clusters (11") of absorbent material corresponding to said porous cavities, such that an absorbent core is formed comprising said top bottom layer (8), said first absorbent material, said intermediate layer (9), said second absorbent material, and said top layer (7);

xiv. optionally joining an acquisition distribution layer to said absorbent core, typically the body facing surface of said top layer (7), and preferably laminating the absorbent core and the acquisition distribution layer between a liquid pervious topsheet and a liquid impervious backsheet;

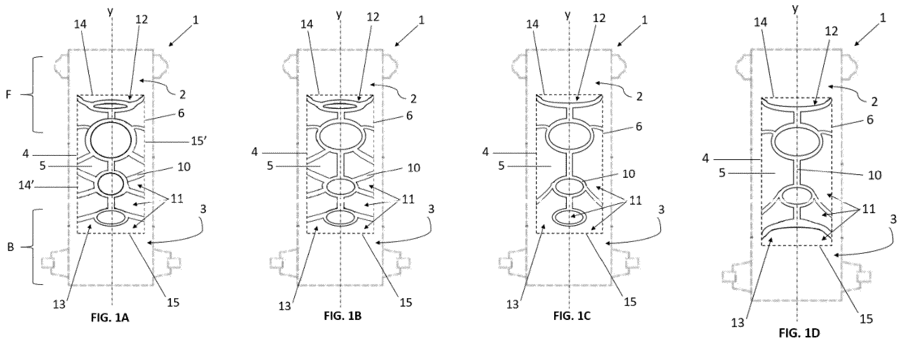

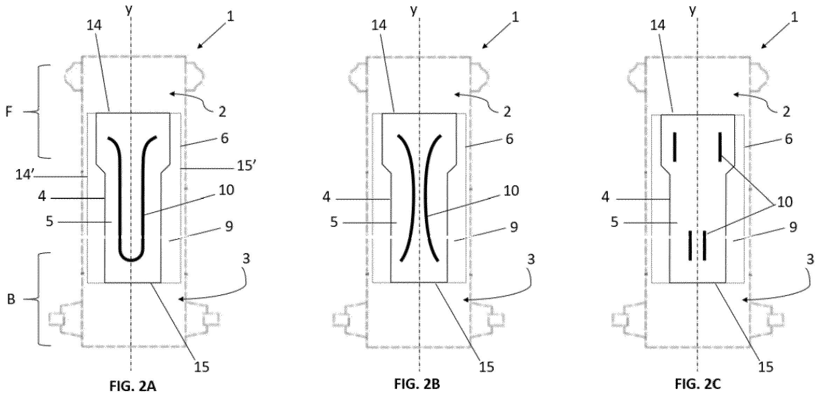

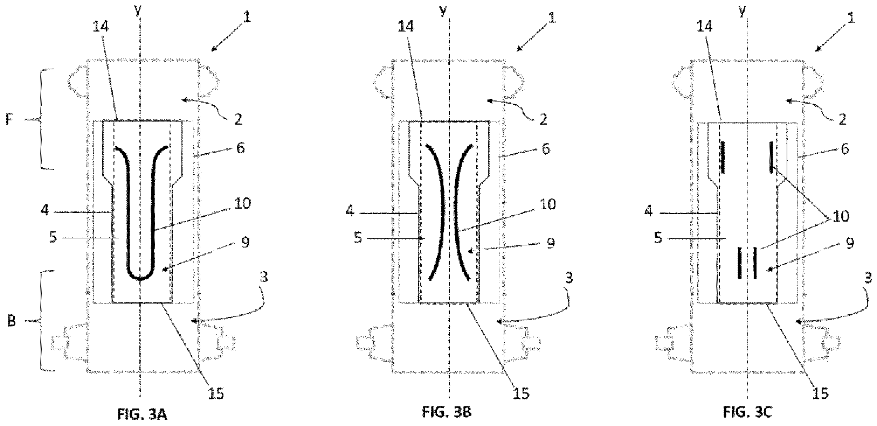

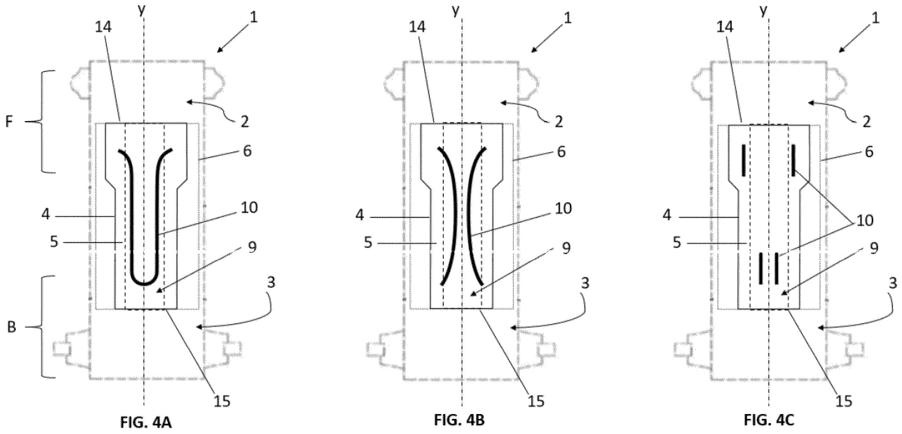

wherein the first plurality of clusters (11') and the second plurality of clusters (11") are substantially congruent or substantially complementary, preferably substantially superpose each other (typically when seen from a planar view generally being a plane formed by the longitudinal axis y and a transverse axis substantially perpendicular thereto as more generally exemplified in Figs. 1-4). Without wishing to be bound by theory, substantially congruent arrangements allow to not only improve core stability but in essence multiply the absorptive retention capacity up to double; whilst substantially complementary (i.e. first plurality of clusters (11') skewed from the second plurality of clusters (11") when seen from a planar view) allow to not only improve core stability but also reduce its overall thickness.

In an embodiment, the first plurality of clusters are mutually separated from the second plurality of clusters by said intermediate layer (9). This arrangement advantageously allows for a separation in the thickness direction that complements the compartments in a width direction to provide additional core stability.

AUL (Absorbency Under Load, 0.7 psi):

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as WO. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0.7psig/g=Wb-Wa/Wa-W0$$

AUL 0.3 psi and 0.5 psi are measured similarly at the appropriate lower pressure.

Absorbtion speed (vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm $\times$ 32 mm, Teflon covered.
4. Stopwatch.
5. Balance, accurate to $\pm$ 0.01 g.
6. Saline solution, 0.9%.

7. Weighing paper.

8. Room with standard condition atmosphere: Temp = 23°C ± 1°C and Relative Humidity = 50% ± 2%.

Test procedure:

1. Measure 50 g ± 0.01 g of saline solution into the 100 ml beaker.

2. Place the magnetic stir bar into the beaker.

3. Program the magnetic stir plate to 600 revolutions per minute.

4. Place the beaker on the center of the magnetic stir plate such that the magnetic stir bar is activated. The bottom of the vortex should be near the top of the stir bar.

5. Weigh out 2 g ± 0.01 g of the superabsorbent material to be tested on weighing paper.

6. While the saline solution is being stirred, pour the superabsorbent material to be tested into the saline solution and start the stopwatch. The superabsorbent material to be tested should be added to the saline solution between the center of the vortex and the side of the beaker.

7. Stop the stopwatch when the surface of the saline solution becomes flat, and record the time.

8. The time, recorded in seconds, is reported as the Vortex Time.

It is supposed that the present invention is not restricted to any form of realization described previously and that some modifications can be added to the presented example of fabrication without reappraisal of the appended claims.

## Claims

1. An apparatus (100) for manufacturing an absorbent article, said apparatus comprising:

   a supporting member (101) for supporting a first sheet material (8) along a surface thereof, wherein the surface of said supporting member (101) is provided with at least one suction zone and at least one non-suction zone;
   a first application unit (102) configured for applying a first absorbent material via an airstream on said first sheet material (8) on the supporting member (101); said first absorbent material being applied such that said first absorbent material is located on a portion of the first sheet material (8) corresponding to the at least one suction zone, and wherein substantially no absorbent material is present on other one or more portions of the first sheet material (8) corresponding to the at least one non-suction zone on at least one first attachment portion (A');
   a first sheet feed unit configured for applying a second sheet material (9) on top of the first absorbent material on the first sheet material (8);
   optionally a first attachment unit (103) configured for attaching said first sheet material (8) to said second sheet material (9) at least in the at least one first attachment portion (A');
   a second application unit (104) configured for applying a second absorbent material via an airstream on said second sheet material (9) and/or first absorbent material; said second absorbent material being applied such that said second absorbent material is located on a portion of the second sheet material (9), and wherein substantially no absorbent material is present on other one or more portions of the second sheet material (9) on at least one second attachment portion (A");
   a second sheet feed unit configured for applying a third sheet material (7) on top of the second absorbent material on the second sheet material (9);
   a second attachment unit (105) configured for attaching said second sheet material (9) to said third sheet material (7) and/or said first sheet material (8) to said second sheet material (9) at least in the at least one second attachment portion (A");
   wherein the first attachment portion (A') and the second attachment portion (A") are substantially congruent or substantially complementary.

2. An apparatus according to Claim 1, wherein the supporting member (101) is a rotating drum and the at least one non-suction zone comprises at least one elongate zone extending in a circumferential direction of the rotating drum.

3. An apparatus according to Claim 2, wherein the at least one non-suction zone is formed by at least one element being substantially planar with an outer surface of the rotating drum; and wherein the at least one suction zone is formed by at least one cavity comprising a substantially porous base, generally in fluid communication with an under pressure source, that is positioned at a first radial distance from the center of said rotating drum being less than a second radial distance of said outer surface from said center.

4. An apparatus according to any of the preceding Claims, further comprising a removing unit comprising a mechanical removal means configured for locally removing the absorbent material applied on said first and/or second sheet material above the at least one non-suction zone.

5. An apparatus according to Claim 4, wherein the mechanical removal means comprises one or more roller brush or air blower.

**6.** An apparatus according to any of the preceding Claims, wherein the first sheet feed unit is positioned between the first application unit (102) and the second application unit (104).

**7.** An apparatus according to any of the preceding Claims, further comprising a first adhesive unit (106) arranged to apply an adhesive pattern on a surface of the second sheet material (9) facing the first absorbent material; and a second adhesive unit (107) arranged to apply an adhesive pattern on a surface of the third sheet material (7) facing the second absorbent material; said first and second adhesive units being positioned upstream of said first and second attachment units respectively.

**8.** An apparatus according to any of the preceding Claims, wherein the first and/or second attachment units (103, 105) comprise a pressure means, such as a pressure roller, arranged to provide an adhering force to join the first, second, and/or third sheet materials respectively; and preferably wherein the first attachment unit (103) comprises a single pressure means, and the second attachment unit (105) comprises a plurality of pressure means.

**9.** An apparatus according to any of the preceding Claims, wherein the first attachment unit (103) is positioned between the first application unit (102) and the second application unit (104), and downstream of the first sheet feed unit.

**10.** A method for manufacturing an absorbent article, said method comprising the steps of:

guiding a first sheet material (8) along a supporting member (101), wherein a surface of said supporting member is provided with a pattern with at least one suction zone and at least one non-suction zone;
applying a first absorbent material via an airstream on said first sheet material (8) on the supporting member, said first absorbent material being applied such that said first absorbent material is located on a portion of the first sheet material (8) corresponding to the at least one suction zone, and wherein substantially no absorbent material is present on other one or more portions of the first sheet material (8) corresponding to the at least one non-suction zone on at least one first attachment portion (A');
applying a second sheet material (9) on top of the first absorbent material on the first sheet material (8); wherein one of said first and second sheet material is a bottom layer of a core wrap substrate (6), and the other one is an intermediate layer;
joining said first sheet material (8) to said second sheet material (9) at least in the at least one first attachment portion (A'), and such that at least one attachment zone is formed;
applying a second absorbent material via an airstream on said second sheet material (9) and/or first absorbent material; said second absorbent material being applied such that said second absorbent material is located on a portion of the second sheet material (9), and wherein substantially no absorbent material is present on other one or more portions of the second sheet material (9) on at least one second attachment portion (A");
applying a third sheet material (7) on top of the second absorbent material on the second sheet material (9); wherein one of said second and third sheet material is an intermediate layer, and the other one is a top layer of a core wrap substrate (6);
joining said second sheet material (9) to said third sheet material (7) at least in the at least one second attachment portion (A"), and such that at least one attachment zone is formed;
wherein the first attachment portion (A') and the second attachment portion (A") are substantially congruent or substantially complementary.

**11.** A method according to Claim 10, further comprising the step of locally removing the absorbent material applied on said first and/or second sheet material, preferably by a mechanical removal means preferably comprising one or more roller brush or air blower.

**12.** A method according to Claims 10 to 11, further comprising the step of applying a first adhesive pattern on a surface of the second sheet material (9) facing the first absorbent material; and applying an second adhesive pattern on a surface of the third sheet material (7) facing the second absorbent material, preferably wherein the first and second patterns are substantially the same or different.

**13.** A method according to Claims 10 to 12, wherein the joining steps comprise the step of applying a pressure and/or adhering force to join the first, second, and/or third sheet materials respectively, substantially concurrently with a suction force within the at least one suction zone typically such to combine a downward push with a substantially simultaneous downward pull wherein downward is the direction from the position at which pressure is applied to or towards the supporting member.

**14.** A method according to Claims 10 to 13, wherein the pattern comprises a plurality, preferably more than 4, even more preferably from 5 to 20, of spaced apart suction zones, typically in the form of distinct cavities, and at least one non-suction zone, such that a plurality of clusters of absorbent material are formed typically after respective application steps.

15. A method according to Claims 10 to 14, wherein the steps are sequential.


**Patentansprüche**

1. Einrichtung (100) zum Herstellen eines absorbierenden Artikels, die Einrichtung umfassend:

   ein Stützelement (101) zum Stützen eines ersten Lagenmaterials (8) entlang einer Oberfläche davon, wobei die Oberfläche des Stützelements (101) mit mindestens einer Saugzone und mindestens einer Nicht-Saugzone versehen ist;
   eine erste Auftrageinheit (102), die zum Auftragen eines ersten absorbierenden Materials über einen Luftstrom auf das erste Lagenmaterial (8) auf dem Stützelement (101) konfiguriert ist; wobei das erste absorbierende Material derart aufgebracht ist, dass es sich auf einem Abschnitt des ersten Lagenmaterials (8) befindet, der der mindestens einen Saugzone entspricht, und wobei auf einem oder mehreren anderen Abschnitten des ersten Lagenmaterials (8), die der mindestens einen Nicht-Saugzone auf mindestens einem ersten Befestigungsabschnitt (A') entsprechen, im Wesentlichen kein absorbierendes Material vorhanden ist;
   eine erste Lagenzufuhreinheit, die zum Aufbringen eines zweiten Lagenmaterials (9) auf das erste absorbierende Material auf dem ersten Lagenmaterial (8) konfiguriert ist;
   optional eine erste Befestigungseinheit (103), die zum Befestigen des ersten Lagenmaterials (8) an dem zweiten Lagenmaterial (9) mindestens in dem mindestens einen ersten Befestigungsabschnitt (A') konfiguriert ist;
   eine zweite Auftrageinheit (104), die zum Auftragen eines zweiten absorbierenden Materials über einen Luftstrom auf das zweite Lagenmaterial (9) und/oder das erste absorbierende Material konfiguriert ist; wobei das zweite absorbierende Material derart aufgebracht ist, dass es sich auf einem Abschnitt des zweiten Lagenmaterials (9) befindet, und wobei auf einem oder mehreren anderen Abschnitten des zweiten Lagenmaterials (9) auf mindestens einem zweiten Befestigungsabschnitt (A'') im Wesentlichen kein absorbierendes Material vorhanden ist;
   eine zweite Lagenzufuhreinheit, die zum Aufbringen eines dritten Lagenmaterials (7) auf das zweite absorbierende Material auf dem zweiten Lagenmaterial (9) konfiguriert ist;
   eine zweite Befestigungseinheit (105), die zum Befestigen des zweiten Lagenmaterials (9) an dem dritten Lagenmaterial (7) und/oder des ersten Lagenmaterials (8) an dem zweiten Lagenmaterial (9) mindestens in dem mindestens einen zweiten Befestigungsabschnitt (A'') konfiguriert ist;
   wobei der erste Befestigungsabschnitt (A') und der zweite Befestigungsabschnitt (A'') im Wesentlichen deckungsgleich oder im Wesentlichen komplementär sind.

2. Einrichtung nach Anspruch 1, wobei das Stützelement (101) eine rotierende Trommel ist und die mindestens eine Nicht-Saugzone mindestens eine längliche Zone umfasst, die sich in einer Umfangsrichtung der rotierenden Trommel erstreckt.

3. Einrichtung nach Anspruch 2, wobei die mindestens eine Nicht-Saugzone durch mindestens ein Element ausgebildet wird, das im Wesentlichen eben mit einer Außenoberfläche der rotierenden Trommel ist; und wobei die mindestens eine Saugzone durch mindestens einen Hohlraum ausgebildet wird, umfassend eine im Wesentlichen poröse Basis, die im Allgemeinen in Fluidkommunikation mit einer Unterdruckquelle steht, die in einem ersten radialen Abstand von der Mitte der rotierenden Trommel positioniert ist, der geringer als ein zweiter radialer Abstand der Außenoberfläche von der Mitte ist.

4. Einrichtung nach einem der vorstehenden Ansprüche, ferner umfassend eine Entfernungseinheit, umfassend ein mechanisches Entfernungsmittel, das zum lokalen Entfernen des absorbierenden Materials konfiguriert ist, das auf dem ersten und/oder dem zweiten Lagenmaterial über der mindestens einen Nicht-Saugzone aufgebracht ist.

5. Einrichtung nach Anspruch 4, wobei die mechanischen Entfernungsmittel eine oder mehrere Walzenbürsten oder Luftgebläse umfassen.

6. Einrichtung nach einem der vorstehenden Ansprüche, wobei die erste Lagenzufuhreinheit zwischen der ersten Auftragseinheit (102) und der zweiten Auftragseinheit (104) positioniert ist.

7. Einrichtung nach einem der vorstehenden Ansprüche, ferner umfassend eine erste Klebeeinheit (106), die angeordnet ist, um ein Klebemuster auf einer Oberfläche des zweiten Lagenmaterials (9) aufzutragen, die dem ersten absorbierenden Material zugewandt ist; und eine zweite Klebeeinheit (107), die angeordnet ist, um ein Klebemuster auf einer Oberfläche des dritten Lagenmaterials (7) aufzutragen, die dem zweiten absorbierenden Material zugewandt ist; wobei die erste und die zweite Klebeeinheit vor der ersten beziehungsweise der zweiten Befestigungseinheit positioniert sind.

8. Einrichtung nach einem der vorstehenden Ansprüche, wobei die erste und/oder zweite Befestigungseinheit (103, 105) ein Druckmittel, wie eine Druckrolle, umfasst, das angeordnet ist, um eine Haftkraft bereitzustellen, um das erste, zweite und/oder dritte Lagenmaterial jeweils zu verbinden; und vorzugsweise wobei die erste Befestigungseinheit (103) ein einzelnes Druckmittel umfasst und die zweite Befestigungseinheit (105) eine Vielzahl von Druckmitteln umfasst.

9. Einrichtung nach einem der absorbierenden Ansprüche, wobei die erste Befestigungseinheit (103) zwischen der ersten Aufbringungseinheit (102) und der zweiten Aufbringungseinheit (104) und stromabwärts der ersten Lagenzufuhreinheit positioniert ist.

10. Verfahren zum Herstellen eines absorbierenden Artikels, das Verfahren umfassend die folgenden Schritte: Führen eines ersten Lagenmaterials (8) entlang eines Stützelements (101), wobei eine Oberfläche des Stützelements mit einem Muster mit mindestens einer Saugzone und mindestens einer Nicht-Saugzone versehen ist;

    Aufbringen eines ersten absorbierenden Materials über einen Luftstrom auf das erste Lagenmaterial (8) auf dem Stützelement, wobei das erste absorbierende Material derart aufgebracht ist, dass es sich auf einem Abschnitt des ersten Lagenmaterials (8) befindet, der der mindestens einen Saugzone entspricht, und wobei auf einem oder mehreren anderen Abschnitten des ersten Lagenmaterials (8), die der mindestens einen Nicht-Saugzone auf mindestens einem ersten Befestigungsabschnitt (A') entsprechen, im Wesentlichen kein absorbierendes Material vorhanden ist;
    Aufbringen eines zweiten Lagenmaterials (9) auf das erste absorbierende Material auf dem ersten Lagenmaterial (8); wobei eines von dem ersten und dem zweiten Lagenmaterial eine untere Schicht eines Kernumhüllungssubstrats (6) ist und das andere eine Zwischenschicht ist;
    Verbinden des ersten Lagenmaterials (8) mit dem zweiten Lagenmaterial (9) mindestens in dem mindestens einen ersten Befestigungsabschnitt (A') und derart, dass mindestens eine Befestigungszone ausgebildet wird;
    Aufbringen eines zweiten absorbierenden Materials über einen Luftstrom auf das zweite Lagenmaterial (9) und/oder das erste absorbierende Material; wobei das zweite absorbierende Material derart aufgebracht ist, dass es sich auf einem Abschnitt des zweiten Lagenmaterials (9) befindet, und wobei auf einem oder mehreren anderen Abschnitten des zweiten Lagenmaterials (9) auf mindestens einem zweiten Befestigungsabschnitt (A") im Wesentlichen kein absorbierendes Material vorhanden ist;
Aufbringen eines dritten Lagenmaterials (7) auf das zweite absorbierende Material auf dem zweiten Lagenmaterial (9); wobei eines von dem zweiten und dem dritten Lagenmaterial eine Zwischenschicht ist und das andere eine Deckschicht eines Kernumhüllungssubstrats (6) ist;
Verbinden des zweiten Lagenmaterials (9) mit dem dritten Lagenmaterial (7) mindestens in dem mindestens einen zweiten Befestigungsabschnitt (A"), und derart, dass mindestens eine Befestigungszone ausgebildet wird;
wobei der erste Befestigungsabschnitt (A') und der zweite Befestigungsabschnitt (A") im Wesentlichen deckungsgleich oder im Wesentlichen komplementär sind.

11. Verfahren nach Anspruch 10, ferner umfassend den Schritt des lokalen Entfernens des absorbierenden Materials, das auf das erste und/oder das zweite Lagenmaterial aufgebracht ist, vorzugsweise durch ein mechanisches Entfernungsmittel, vorzugsweise umfassend eine oder mehrere Walzenbürsten oder einen Luftbläser.

12. Verfahren nach den Ansprüchen 10 bis 11, ferner umfassend den Schritt des Aufbringens eines ersten Klebemusters auf eine Oberfläche des zweiten Lagenmaterials (9), die dem ersten absorbierenden Material zugewandt ist; und Aufbringen eines zweiten Klebemusters auf einer Oberfläche des dritten Lagenmaterials (7), die dem zweiten absorbierenden Material zugewandt ist, wobei das erste und das zweite Muster vorzugsweise im Wesentlichen gleich oder unterschiedlich sind.

13. Verfahren nach den Ansprüchen 10 bis 12, wobei die Verbindungsschritte den Schritt des Aufbringens eines Drucks und/oder einer Haftkraft umfassen, um das erste, das zweite und/oder das dritte Lagenmaterial zu verbinden, im Wesentlichen gleichzeitig mit einer Saugkraft innerhalb der mindestens einen Saugzone, typischerweise derart, dass ein Abwärtsdrücken mit einem im Wesentlichen gleichzeitigen Abwärtsziehen kombiniert wird, wobei "abwärts" die Richtung von der Position ist, an der Druck auf das Stützelement oder zu diesem hin aufgebracht ist.

14. Verfahren nach den Ansprüchen 10 bis 13, wobei das Muster eine Vielzahl, vorzugsweise mehr als 4, noch mehr bevorzugt 5 bis 20, von voneinander beabstandeten Saugzonen, typischerweise in Form von einzelnen Hohlräumen, und mindestens eine Nicht-Saugzone derart umfasst, dass typischerweise nach den jeweiligen Anwendungsschritten eine Vielzahl von Clustern aus absorbierendem Material ausgebildet werden.

15. Verfahren nach den Ansprüchen 10 bis 14, wobei die Schritte aufeinander folgen.

## Revendications

1. Appareil (100) permettant de fabriquer un article absorbant, ledit appareil comprenant :

un élément de support (101) permettant de supporter un premier matériau en feuille (8) le long d'une surface de celui-ci, dans lequel la surface dudit élément de support (101) est pourvue d'au moins une zone d'aspiration et d'au moins une zone sans aspiration ;
une première unité d'application (102) conçue pour l'application d'un premier matériau absorbant par l'intermédiaire d'un courant d'air sur ledit premier matériau en feuille (8) sur l'élément de support (101) ; ledit premier matériau absorbant étant appliqué de telle sorte que ledit premier matériau absorbant est localisé sur une partie du premier matériau en feuille (8) correspondant à l'au moins une zone d'aspiration, et dans lequel sensiblement aucun matériau absorbant n'est présent sur une ou plusieurs autres parties du premier matériau en feuille (8) correspondant à l'au moins une zone sans aspiration sur au moins une première partie d'attachement (A') ;
une première unité d'alimentation de feuille conçue pour l'application d'un deuxième matériau en feuille (9) au-dessus du premier matériau absorbant sur le premier matériau en feuille (8) ;
facultativement une première unité d'attachement (103) conçue pour l'attachement dudit premier matériau en feuille (8) audit deuxième matériau en feuille (9) au moins dans l'au moins une première partie d'attachement (A') ;
une seconde unité d'application (104) conçue pour l'application d'un second matériau absorbant par l'intermédiaire d'un courant d'air sur ledit deuxième matériau en feuille (9) et/ou le premier matériau absorbant ; ledit second matériau absorbant étant appliqué de telle sorte que ledit second matériau absorbant est localisé sur une partie du deuxième matériau en feuille (9), et dans lequel sensiblement aucun matériau absorbant n'est présent sur une ou plusieurs autres parties du deuxième matériau en feuille (9) sur au moins une seconde partie d'attachement (A") ;
une seconde unité d'alimentation de feuille conçue pour l'application d'un troisième matériau en feuille (7) au-dessus du second matériau absorbant sur le deuxième matériau en feuille (9) ;
une seconde unité d'attachement (105) conçue pour l'attachement dudit deuxième matériau en feuille (9) audit troisième matériau en feuille (7) et/ou dudit premier matériau en feuille (8) audit deuxième matériau en feuille (9) au moins dans l'au moins une seconde partie d'attachement (A") ;
dans lequel la première partie d'attachement (A') et la seconde partie d'attachement (A") sont sensiblement congruentes ou sensiblement complémentaires.

2. Appareil selon la revendication 1, dans lequel l'élément de support (101) est un tambour en rotation et l'au moins une zone sans aspiration comprend au moins une zone allongée s'étendant dans une direction circonférentielle du tambour en rotation.

3. Appareil selon la revendication 2, dans lequel l'au moins une zone sans aspiration est formée par au moins un élément étant sensiblement plan avec une surface externe du tambour en rotation ; et dans lequel l'au moins une zone d'aspiration est formée par au moins une cavité comprenant une base sensiblement poreuse, généralement en communication fluidique avec une source de dépression, qui est positionnée à une première distance radiale par rapport au centre dudit tambour en rotation inférieure à une seconde distance radiale de ladite surface externe par rapport audit centre.

4. Appareil selon l'une quelconque des revendications précédentes, comprenant en outre une unité de retrait comprenant un moyen de retrait mécanique conçu pour retirer localement le matériau absorbant appliqué sur lesdits premier et/ou deuxième matériaux en feuille au-dessus de l'au moins une zone sans aspiration.

5. Appareil selon la revendication 4, dans lequel le moyen de retrait mécanique comprend une ou plusieurs brosses à rouleaux ou soufflantes d'air.

6. Appareil selon l'une quelconque des revendications précédentes, dans lequel la première unité d'alimentation de feuille est positionnée entre la première unité d'application (102) et la seconde unité d'application (104).

7. Appareil selon l'une quelconque des revendications précédentes, comprenant en outre une première unité d'adhésif (106) agencée pour appliquer un motif adhésif sur une surface du deuxième matériau en feuille (9) faisant face vers le premier matériau absorbant ; et une seconde unité d'adhésif (107) agencée pour appliquer un motif adhésif sur une surface du troisième matériau en feuille (7) faisant face vers le second matériau absorbant ; lesdites première et seconde unités d'adhésif étant positionnées en amont desdites première et seconde unités d'attachement respectivement.

8. Appareil selon l'une quelconque des revendications précédentes, dans lequel les première et/ou seconde unités d'attachement (103, 105) comprennent un moyen de pression, tel qu'un rouleau de pression, agencé pour fournir une force d'adhérence pour joindre les premier, deuxième et/ou troisième matériaux en feuille respectivement ; et de préférence dans lequel la première unité d'attachement (103) comprend un unique moyen de pression, et la seconde unité d'attachement (105) comprend une pluralité de moyens de pression.

9. Appareil selon l'une quelconque des revendications précédentes, dans lequel la première unité d'attachement (103) est positionnée entre la première unité d'application (102) et la seconde unité d'application (104), et en aval de la première unité d'alimentation de feuille.

10. Procédé permettant de fabriquer un article absorbant, ledit procédé comprenant les étapes consistant à : guider un premier matériau en feuille (8) le long d'un élément de support (101), dans lequel une surface dudit élément de support est pourvue d'un motif avec au moins une zone d'aspiration et au moins une zone sans aspiration ;

appliquer un premier matériau absorbant par l'intermédiaire d'un courant d'air sur ledit premier matériau en feuille (8) sur l'élément de support, ledit premier matériau absorbant étant appliqué de telle sorte que ledit premier matériau absorbant est localisé sur une partie du premier matériau en feuille (8) correspondant à l'au moins une zone d'aspiration, et dans lequel sensiblement aucun matériau absorbant n'est présent sur une ou plusieurs autres parties du premier matériau en feuille (8) correspondant à l'au moins une zone sans aspiration sur au moins une première partie d'attachement (A') ; appliquer un deuxième matériau en feuille (9) au-dessus du premier matériau absorbant sur le premier matériau en feuille (8) ; dans lequel l'un desdits premier et deuxième matériaux en feuille est une couche inférieure d'un substrat d'enveloppe d'âme (6), et l'autre est une couche intermédiaire ; joindre ledit premier matériau en feuille (8) audit deuxième matériau en feuille (9) au moins dans l'au moins une première partie d'attachement (A'), et de telle sorte qu'au moins une zone d'attachement est formée ; appliquer un second matériau absorbant par l'intermédiaire d'un courant d'air sur ledit deuxième matériau en feuille (9) et/ou le premier matériau absorbant ; ledit second matériau absorbant étant appliqué de telle sorte que ledit second matériau absorbant est localisé sur une partie du deuxième matériau en feuille (9), et dans lequel sensiblement aucun matériau absorbant n'est présent sur une ou plusieurs autres parties du deuxième matériau en feuille (9) sur au moins une seconde partie d'attachement (A") ; appliquer un troisième matériau en feuille (7) au-dessus du second matériau absorbant sur le deuxième matériau en feuille (9) ; dans lequel l'un desdits deuxième et troisième matériaux en feuille est une couche intermédiaire, et l'autre est une couche supérieure d'un substrat d'enveloppe d'âme (6) ; joindre ledit deuxième matériau en feuille (9) audit troisième matériau en feuille (7) au moins dans l'au moins une seconde partie d'attachement (A"), et de telle sorte qu'au moins une zone d'attachement est formée ; dans lequel la première partie d'attachement (A') et la seconde partie d'attachement (A") sont sensiblement congruentes ou sensiblement complémentaires.

11. Procédé selon la revendication 10, comprenant en outre l'étape consistant à retirer localement le matériau absorbant appliqué sur lesdits premier et/ou deuxième matériaux en feuille, de préférence par un moyen de retrait mécanique comprenant de préférence une ou plusieurs brosses à rouleaux ou soufflantes d'air.

12. Procédé selon les revendications 10 à 11, comprenant en outre l'étape consistant à appliquer un premier motif adhésif sur une surface du deuxième matériau en feuille (9) faisant face vers le premier matériau absorbant ; et à appliquer un second motif adhésif sur une surface du troisième matériau en feuille (7) faisant face vers le second matériau absorbant, de préférence dans lequel les premier et second motifs sont sensiblement identiques ou différents.

13. Procédé selon les revendications 10 à 12, dans lequel les étapes de jonction comprennent l'étape consistant à appliquer une pression et/ou une force d'adhérence pour joindre les premier, deuxième et/ou troisième matériaux en feuille, sensiblement en même temps qu'une force d'aspiration au sein de l'au moins une zone d'aspiration typiquement de façon à combiner une poussée vers le bas avec un traction vers le bas sensiblement simultanée dans lequel vers le bas est la direction à partir de la position vers laquelle la pression est appliquée ou en direction de l'élément de support.

14. Procédé selon les revendications 10 à 13, dans lequel le motif comprend une pluralité, de préférence plus de 4, même plus préférablement de 5 à 20, de zones d'aspiration espacées, typiquement sous la forme de cavités distinctes, et au moins une zone sans aspiration, de telle sorte qu'une pluralité de grappes de matériau absorbant sont formées typiquement après les étapes d'application respectives.

15. Procédé selon les revendications 10 à 14, dans lequel les étapes sont séquentielles.

FIG. 1A

FIG. 1B

FIG. 1C

FIG. 1D

FIG. 2A

FIG. 2B

FIG. 2C

FIG. 3A

FIG. 3B

FIG. 3C

FIG. 4A

FIG. 4B

FIG. 4C

FIG. 5A

FIG. 5B

FIG. 6A

FIG. 6B

FIG. 6C

FIG. 7A

FIG. 7B

FIG. 7C

FIG. 7D

**FIG. 8**

FIG. 9A

FIG. 9B

FIG. 9C

FIG. 9D

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012170778 A, Rosati
- WO 2012170779 A
- WO 2012170781 A
- WO 20121708008 A
- EP 3342386 A
- WO 2018172860 A
- EP 3453368 A
- US 20160045379 A1
- WO 2018172860 A1
- US 20080312618 A1
- US 3485706 A, Evans
- US 20090306290 A